# EUROPEAN PATENT APPLICATION

(11) **EP 0 649 856 A1**
(43) Date of publication of application: **26.04.1995**
(21) Application number: 93810733.1
(22) Date of filing: 19.10.1993
(51) Int. Cl.: C07K 1/113, C07K 14/815

(54) **Process for the production of correct folded protein**

(71) Applicant: CIBA-GEIGY AG, CH-4002 Basel (CH); UCP GEN-PHARMA AG, CH-8044 Zürich (CH)
(72) Inventor: Chang,Jui Yoa,Dr.,, 4416 Bubendorf (CH)

(57) **Abstract**

The invention relates to a process for the preparation of a biologically active and correctly folded protein, using a protein disulfide exchange enzyme and a redox system containing a sulfhydryl and a disulfide compound.

## Description

The invention relates to a process for the preparation of a biologically active and correctly folded protein, using a protein disulfide exchange enzyme and a redox system containing a sulfhydryl and a disulfide compound.

Correct folding of genetically engineered proteins is a major issue in protein science. The problem is particularly acute in the case of disulfide-containing proteins, which often need to be denatured and refolded to become active. Disulfide formation is an event of post-translational modification. It follows the pathway of protein folding and usually occurs faithfully *in vivo*. However, in the *in vitro* renaturation experiments, many proteins are recovered in poor yields and some do not fold into native structures at all. To overcome this problem, a number of compounds, such as the mixture of reduced / oxidized glutathione (Creighton, Methods Enzymol. **131** (1986), 83-106) and protein disulfide isomerase (PDI) (Morin, J. E. & Dixon, J. E. Methods Enzymol. **113** (1985), 541-547) have been routinely used to promote the formation of disulfides. But despite their widespread use, the precise mechanism of their function remains undefined and their applications have been conducted in a manner of trial and error. Therefore, the efficiencies of *in vitro* folding documented so far are nowhere near those observed *in vivo*.

A number of publications have appeared which report refolding attempts for individual proteins produced in bacterial hosts, or which are otherwise in a denatured or non-native form. Formation of a dimeric, biologically active human colony stimulating factor-1 (CSF-1) after expression in *E. coli* is described in WO 88/8003 and by Halenbeck et al. Biotechnologie, **7** (1989), 710-715. The procedures described involve the steps of initial solubilization of CSF-1 monomers isolated from inclusion bodies under reducing conditions in a chaotropic environment comprising urea or guanidine hydrochloride, refolding which is achieved by stepwise dilution of the chaotropic agents, and final oxidation of the refolded molecule in the presence of a redox-system. In WO 88/8849 a process for recovering recombinant interleukin-2 (IL-2) is disclosed, characterized in that IL-2 isolated from refractile bodies is denatured under reducing conditions with 6 M guanidine hydrochloride, the soluble IL-2 is oxidized by a controlled oxidation in the presence of Cu²⁺ions, and the oxidized IL-2 refolded by reducing the concentration of the denaturant in the solution. Interleukin-2 and interferon-β have been refolded using SDS for solubilization and Cu²⁺ions as oxidation promoters of the fully reduced protein (US-A-4 572 798). The process for isolating recombinant refractile proteins as described in US-A-4 620 948 involves strong denaturing agents to solubilize the proteins, reducing conditions to facilitate correct folding and denaturant replacement in presence of air or other oxidizing agents to reform the disulfide bonds. A method for renaturing unfolded proteins including cytochrome c, ovalbumin and trypsin inhibitor by reversibly binding of the denatured protein to a solid matrix and stepwise renaturing it by diluting the denaturant is disclosed in WO 86/5809. The foregoing references are merely representative of a huge amount of literature dealing with the refolding of non-native proteins derived from different sources. The man skilled in the art on the other hand knows that the success of refolding experiments cannot be predicted. Unsucessful experiments are usually not reported. There is no certainty that anyone of the reported refolding conditions would work at all with a given denatured protein containing several cysteine residues and therefore, a number of intramolecular disulfide bonds, which are required for activity.

It is therefore an object of the invention to provide a process for the production of a correct folded, disulfide bonds containing protein, or a salt thereof, characterized in that the denatured protein is treated with a buffer comprising a RSH/RS-SR mixture and a protein disulfide exchange enzyme, wherein the radical R effects a redox potential of RSH from -0.20 V to -0.23 V.

A correct folded protein is in the native conformation and shows a biological activity like the enzymatic activity or the binding property of the native protein.

With the inventive process, the time for the quantitative *in vitro* folding into the native conformation is less than 2 minutes and preferred less than 1 minute.

For example, with the inventive process it is possible to shorten the time required for *in vitro* folding of hirudin core domain from 10 hours to 30 seconds. This efficiency of *in vitro* folding does not differ significantly from what occur *in vivo*, which has been shown to accomplish within 2 min (Freedman, Trends Biochem. Sci. **9** (1984), 438-441).

The folding pathway of proteins containing disulfide bonds, especially intramolecular disulfide bonds, is usually characterized by an initial non-specific packing, followed by consolidation (reorganization) of partially packed intermediates to reach the native structure. The structure and number of the intermediates can be analyzed in stop/go folding experiments of acid-trapped intermediates (Chatrenet & Chang, Biol. Chem. **267** (1992), 3038-3043).

In the case of hirudin, the course of packing involves a sequential flow of the unfolded sample [R], containing no disulfide bonds, through equilibrated isomers with one disulfide bond [I] and equilibrated isomers with two disulfide bonds [II] to equilibrated (scrambled) species [III] wherein three disulfide bonds are formed. In the (scrambled) species [III], some non-native disulfide bonds are present. The process of consolidation is characterized as scrambled species reorganize by reshuffling the non-native disulfide bonds to attain the correctly folded native species [N]. 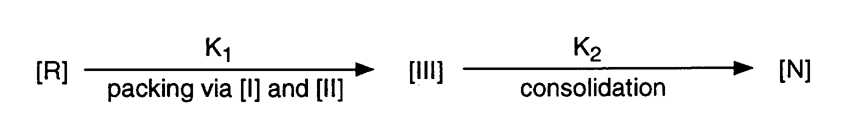
The inventive process is therefore applicable, e.g., to proteins wherein the folding mechanism is characterized by an intermediate comprising incorrect formed disulfide bonds. Preferred are proteins, wherein the folding mechanism is characterized by
a) a first group of intermediates in which not all possible disulfide bonds are formed; and
b) a second group of intermediates in which all possible disulfide bonds are formed but one or more of the disulfide bonds formed are non-native.

In a more preferred embodiment of the invention the protein is hirudin and especially preferred is the hirudin core domain consisting of amino acid residue 1-49.

Hirudin is a thrombin specific inhibitor originated from leech *Hirudo medicinalis* (Markwardt, Methods Enzymol. **19** (1990), 924-932). The inhibitor contains two functional domains which bind to independent sites of α-thrombin; a compact N-terminal core domain binding to the catalytic site of thrombin and a disordered C-terminal tail which is complementary to the exosite (fibrinogen recognition site) of the enzyme. The core domain (49 amino acids) requires proper folding to maintain its biological function and is stabilized by 3 disulfides (Cys⁶-Cys¹⁴, Cys¹⁶-Cys²⁸ and Cys²²-Cys³⁹) that are closely embedded within the compact core. Recently, the folding pathway of the hirudin core domain has been elucidated by structural analysis and stop/go folding experiments of acid-trapped intermediates (Chatrenet & Chang, Biol. Chem. **267** (1992), 3038-3043).

In recent years cDNAs and synthetic genes coding for hirudin variants have been cloned and expressed in microbial hosts, such as *Escherichia coli* and, in particular, *Saccharomyces cerevisiae* (EP-A-324712, EP-A-341215, EP-A-340170). Although the expression products lack the sulphate monoester group at Tyr⁶³ - and were therefore designated "desulphatohirudins" - they turned out to exhibit essentially the same biological activity as the natural sulphated hirudins.

The term "hirudin" as used in this invention is intended to embrace all desulphatohirudin compounds described in literature or obtainable from a transformed microorganism strain containing DNA which codes for a desulphatohirudin or a derivative thereof. Such hirudins are, for example, desulphatohirudin derivatives HV1, HV2 and HV3 (PA), as well as other hirudin proteins as described e.g. by M. Scharf *et al*. (FEBS Lett., **255** (1989), 105-110) and EP-A-347 376. It is to be understood that hirudin derivatives or shorter fragments having hirudin activity are also covered by the term "hirudin". Such fragments and derivatives are, for example, C-terminally shortened desulphatohirudins.

During the folding of proteins, the speed of disulfide formation and the process of packing (K₁) is drastically accelerated in the presence of a disulfide bond containing compound. Packing leads to the accumulation of scrambled proteins [III]. Therefore, in the presence of disulfides, scrambled species become the most dominant folding intermediates and conversion of the scrambled species to the native structure [N] (consolidation, K₂) become the major rate limiting step of protein folding.

Conversion of scrambled proteins [III] to the native structure [R] requires free thiols as catalyst. When a protein was allowed to fold in the buffer alone, free cysteins of the folding intermediates serve as the thiol catalyst during the early phase of folding. As the folding advances, more cysteins become involved in the disulfide paring and less are available as thiol catalyst.

Therefore, thiols, disulfides and mixtures thereof like oxidized glutathione (GSSG), GSH/GSSG, oxidized trans-4,5-dihydroxy-1,2-dithiane (DTT), β-mercaptoethanol (HSEtOH) and PDI are most commonly used to enhance disulfide formation and reformation during protein folding (table 1).

**Table 1**

| Compound | Redoxpotential E₀ at pH / and 25°C |
|---|---|
| GSH | -0.24 V |
| DTT | -0.33 V |
| HSEtOH | -0.27 to -0.29 V |
| Cys | -0.22 V |

It has been found that the redoxpotential of the RSH compound has a surprisingly large impact on the folding into the native conformation and that the addition of a redoxsystems RSH/RS-SR wherein RSH has a redoxpotential from -0.20 V to -0.23 V substantially decreases the time needed for correct folding of the protein into the native structure. Examples for suitable RSH/RS-SR mixtures are thioredoxin and Cys/Cys-Cys. An especially preferred redox system is Cys/Cys-Cys.

The ratio of RSH to RS-SR is preferably 2 : 1 to 1 : 5 and more preferably about 1 : 1.5 to 1 : 2.5. The total concentration of RSH and RS-SR is preferably 1 to 10 mM and especially preferred is a mixture wherein the concentration of RSH is 2 to 6 mM and the concentration of RS-SR is 1 to 4 mM.

For the consolidation of scrambled proteins [III], to attain the native conformation by reshuffling, protein disulfide exchange enzymes like PDI alone have no effect. For example, scrambled hirudin remains completely stable in the alkaline buffer containing 2 mM or 10 mM of PDI. Surprisingly, in the presence of free thiols, PDI displays an additive effect in enhancing the process of consolidation.

Several protein disulfide exchange enzymes like protein disulfide isomerase (PDI) and other thioredoxin-like proteins are described in the literature (Morin & Dixon, Methods Enzymol. **113** (1985), 541-547; Freedman, Cell **57** (1989), 1069-1072 and Pigiet & Schuster, Proc. Natl. Acad. Sci. U.S.A., **83** (1986) 7643-7647) and are suitable in the process of consolidation. In a preferred embodiment of the invention PDI is used.

The concentration of the protein disulfide isomerases normally is from 5 to 1000 µM, preferred from 10 to 100 µM and more preferred from 20 to 60 µM.

Especially preferred is a process for the production of a correct folded, disulfide bonds containing protein, or a salt thereof, comprising solubilizing said protein in a buffer containing 2 to 6 mM Cys and 1 to 4 mM Cys-Cys with a ratio of Cys:Cys-Cys from 1:1.5 to 1:2.5; and 40 to 60 µM PDI.

All buffer that have no inhibitory effect on the folding of proteins are applicable. Therefore, the buffer is usually cleared from chaotropic agents like urea or guanidine hydrochloride or other folding inhibiting compounds through a desalting step. Common purification procedures are columns equilibrated with NaHCO₃ or Tris base at a certain pH. The pH and temperature are preferably within a range wherein the native form of the protein is stable.

Proteins in a non-native conformation are produced in a manner known per se. They are usually isolated from a genetically engineered host like bacteria and fungi or produced by a protein synthesizer. Reasons for the non-native conformation of heterologous produced proteins are, for example, missing enzymes for post-translational modification like chaperones; conditions that unfold the protein during isolation and purification; or proteins that have to be denatured and refolded to become active.

For the denaturation of proteins all methods are applicable that do not lead to an irreversible denaturation e.g. via cleavage of peptide bonds. Reversible denaturation is achieved for example by the addition of chaotropic agents. Chaotropic agents in suitable concentration are capable of effectively denaturing proteins by changing the spatial configuration of the respective protein through alterations at the surface thereof, either through altering the state of hydration, the solvent environment, or the solvent-surface interaction in aqueous solution and are well known in the art. Examples of such chaotropic agents or denaturants include urea, guanidine hydrochloride, sodium thiocyanate at concentrations in the range of about 4 to 9 M, and detergents such as SDS, which are supplied in concentrations in the order of 0.01 to 2 %. Also, acidification of the aqueous solution, basic conditions, and elevated temperatures are common for denaturing of proteins.

### Brief description of the drawings

In the following experimental part various embodiments of the present invention are described with reference to the accompanying drawings in which:

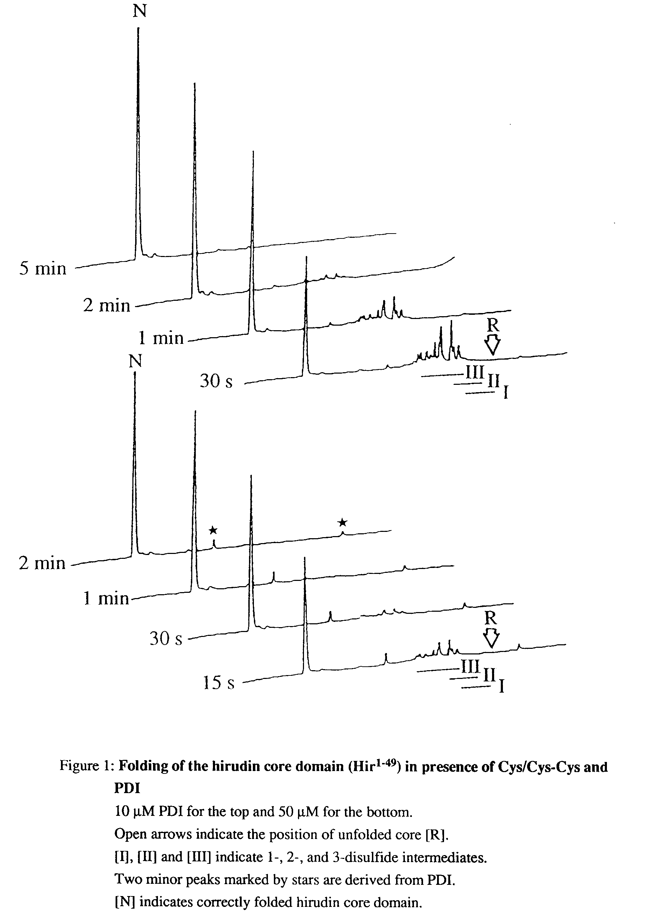

Fig. 1 is a HPLC protocol of the folding of the hirudin core domain (Hir¹⁻⁴⁹) in presence of Cys/Cys-Cys and PDI

### EXPERIMENTAL PROCEDURES

### Example 1: Production and isolation of the hirudin core domain (Hir¹⁻⁴⁹)

Recombinant desulphated hirudin was is isolated from Saccharomyces cerevisiae as described in Meyhack et al. (Thromb. Res. Suppl. VII (1987), 33) (SEQ ID NO:1). The isolated desulphatohirudin is dissolved in 50 mM ammoniumbicarbonate buffer pH 8.0 at a concentration of 5 mg/ml and digested with chymotrypsin (0.25 mg/ml) at room temperature for 16 h. The digestion is terminated by addition of trifluoroacetic acid to a final concentration of 0.8 % and the core domain (Hir¹⁻⁴⁹) is isolated by HPLC.
Condition for HPLC:
Column: Vydac C-18
Solvent A: 0.1 % trifluoroacetic acid in water
Solvent B: 0.1 % trifluoroacetic acid in acetonitrile
Gradient: 10 % B to 48 % B in 30 min at 23°C
Retention time for Hir¹⁻⁴⁹: 14.3 min

### Example 2: Denaturation of the hirudin core domain (Hir¹⁻⁴⁹)

The starting material for the folding experiments, fully reduced / denatured core domain of hirudin [R], is prepared by the following method:

The hirudin core domain from example 1(2 mg/ml) is dissolved in Tris-HCl buffer (0.5 M, pH 8.5) containing 5 M of guanidine chloride (GdmCl) and 30 mM of dithiothreitol. Reduction and denaturation are carried out at 23°C for 90 min. To initiate the folding, the sample is passed through a PD-10 column (Pharmacia) equilibrated in 0.1 M NaHCO₃ buffer (pH 8.3). Desalting takes about 1 min and [R] is collected in 1.1 ml. The collected sample is immediately distributed and diluted to concentrations used in the folding experiments.

### Example 3: Protein folding in the presence of redoxsystems

Each sample contains core domain (Hir¹⁻⁴⁹) in a concentration of 70 mM and a selected concentration of oxidized glutathione (GSSG), Cys-Cys, Cys/Cys-Cys mixture and PDI (the experiment containing the Cys/Cys-Cys mixture additionally contains 4 M NaCl). The folding intermediates are trapped in a time course manner by mixing aliquots of the sample with equal volume of 4% trifluoroacetic acid and are subsequently analyzed by HPLC (see table 2).
Conditions for HPLC:
Column: Vydac C-18
Solvent A: water containing 0.1% trifluoroacetic acid
Solvent B: acetonitrile/water (9:1, by volume) containing 0.1% trifluoroacetic acid
Gradient: 16% -30% solvent B linear in 50 min.
Flow rate: 1 ml/min.

Table 2 shows the amount of correctly folded protein in %

**Table 2**

| Time [min] | GSSG | | | CysCys | | | Cys/CysCys 4 mM/2 mM | PDI 10 µM |
|---|---|---|---|---|---|---|---|---|
| | 0.5 mM | 2 mM | 4 mM | 0.5 mM | 2 mM | 4 mM | | |
| 0 | 0 | 0 | 0 | 0 | 0 | 0 | 0 | 0 |
| 2 | 3 | 5 | 7 | 15 | 25 | 25 | 66 | 2 |
| 5 | 5 | 15 | 12 | 25 | 30 | 30 | 95 | 7 |
| 15 | 18 | 25 | 25 | 50 | 52 | 45 | >99 | 21 |

### Example 4: Effect of PDI on the consolidation of scrambled Hir¹⁻⁴⁹

Scrambled Hir¹⁻⁴⁹ (III) is produced by keeping fully reduced and denatured Hir¹⁻⁴⁹ for 24 h at room temperature in sodium bicarbonate buffer (0.1 M, pH 8.3). PDI is added to a final concentration of 10 µM (70 µM Hir¹⁻⁴⁹).

In a control PDI is replaced by 1 mM Cys.

The folding intermediates are trapped after 30 min by mixing aliquots of the sample with equal volume of 4% trifluoroacetic acid and are subsequently analyzed by HPLC as described in example 3.

In the presence of PDI no Hir¹⁻⁴⁹ in the native conformation is formed, while in the presence of Cys (1 mM) after 30 min <95 % of the Hir¹⁻⁴⁹ is in the native conformation.

### Example 5: Accelerated pathway of hirudin folding

Samples from example 2 are allowed to fold in Tris-HCl buffer (0.2 M, pH 8.5) containing NaCl (4 M), Cys / Cys-Cys (4 mM / 2 mM) and protein disulfide isomerase (PDI; 10 µM and 50 µM). Folding intermediates are trapped by acid (see example 4) and analyzed by HPLC as described in example 3. In the presence of 50 µM of PDI, folding virtually completed within 30 seconds (figure 1).

In figure 1, 10 µM PDI are used for the top series and 50 µM for the bottom series; open arrows indicate the position of unfolded core [R]; stars indicate peaks derived from PDI; [I], [II] and [III] indicate 1-, 2-, and 3-disulfide intermediates; and [N] indicates correctly folded hirudin core domain.

## Claims

1. A process for the production of a correct folded, disulfide bonds containing protein, or a salt thereof, characterized in that the denatured protein is treated with a buffer comprising a RSH/RS-SR mixture and a protein disulfide exchange enzyme, wherein the radical R effects a redox potential of RSH from -0.20 V to -0.23 V.

2. A process according to claim 1, wherein the folding mechanism of the protein is characterized by an intermediate comprising incorrect formed disulfide bonds.

3. A process according to claim 1, wherein the folding mechanism is characterized by
a) a first group of intermediates in which not all possible disulfide bonds are formed; and
b) a second group of intermediates in which all possible disulfide bonds are formed but one or more of the disulfide bonds formed are non-native.

4. A process according to claim 1, wherein the protein is hirudin or a variant thereof.

5. A process according to claim 1, wherein the protein is the hirudin core domain consisting of amino acid residue 1-49.

6. A process according to claim 1, wherein the RSH/RS-SR mixture is selected from the group consisting of thioredoxin and Cys/Cys-Cys.

7. A process according to claim 1, wherein the RSH/RS-SR mixture is Cys/Cys-Cys.

8. A process according to claim 1, wherein the ratio of RSH : RS-SR is 2 : 1 to 1 : 5.

9. A process according to claim 8, wherein the ratio of RSH : RS-SR is about 1 : 1.5 to 1 :2.5.

10. A process according to claim 8, wherein the total concentration of RSH and RS-SR is 1 to 10 mM.

11. A process according to claim 10, wherein the concentration of RSH is 2 to 6 mM and the concentration of RS-SR is 1 to 4 mM.

12. A process according to claim 1, wherein the concentration of PDI is 5 to 1000 µM.

13. A process according to claim 1, wherein the concentration of PDI is 10 to 100 µM.

14. A process according to claim 1, wherein the concentration of PDI is 20 to 60 µM.

15. A process according to claim 1, comprising solubilizing of a protein according to claim 1 in a buffer containing 2 to 6 mM Cys and 1 to 4 mM Cys-Cys with a ratio of Cys:Cys-Cys from 1:1.5 to 1:2.5, and 40 to 60 µM PDI.


SEQUENCE LISTING

(1) GENERAL INFORMATION:

(i) APPLICANT:
(A) NAME: CIBA-GEIGY AG
(B) STREET: Klybeckstr. 141
(C) CITY: Basel
(E) COUNTRY: Switzerland
(F) POSTAL CODE (ZIP): 4002
(G) TELEPHONE: +41 61 69 11 11
(H) TELEFAX: + 41 61 696 79 76
(I) TELEX: 962 991

(A) NAME: UCP GEN PHARMA AG
(B) STREET: Kraftstrasse 6
(C) CITY: Zuerich
(E) COUNTRY: Switzerland
(F) POSTAL CODE (ZIP): 8044

(ii) TITLE OF INVENTION: Process for the production of correct folded
proteins

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:

(A) LENGTH: 64 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION: 1..64
(D) OTHER INFORMATION: /label= HV1
/note= "Hirudin variant HV1"

(ix) FEATURE:
(A) NAME/KEY: Domain
(B) LOCATION: 1..49
(D) OTHER INFORMATION: /label= core
/note= "Hirudin core domain"

(xi) SEQUENCE DESCRIPTION: SEQ ID NO: 1:

Val Val Tyr Thr Asp Cys Thr Glu Ser Gly Gln Asn Leu Cys Leu Cys
1               5                   10                  15

Glu Gly Ser Asn Val Cys Gly Gln Gly Asn Lys Cys Ile Leu Gly Ser
            20                  25                  30

Asp Gly Glu Lys Asn Gln Cys Val Thr Gly Glu Gly Thr Pro Lys Pro
        35                  40                  45

Gln Ser His Asn Asp Gly Asp Phe Glu Glu Ile Pro Glu Glu Tyr Leu
    50                  55                  60